# EUROPEAN PATENT APPLICATION

(11) **EP 4 397 313 A1**
(43) Date of publication of application: **10.07.2024**
(21) Application number: 23834546.6
(22) Date of filing: 31.05.2023
(51) Int. Cl.: A61K 35/60, C11B 3/12, C11B 5/00, A61K 31/355

(54) **METHOD FOR REDUCING OLIGOMER CONTENT IN FISH OIL**

(30) Priority: 07.07.2022 CN 202210802874
(71) Applicant: Kinomega Biopharm Inc., Deyang, Sichuan 618400 (CN)
(72) Inventor: YANG, Guowen, Shifang Deyang, Sichuan 618400 (CN); FAN, Youjun, Shifang Deyang, Sichuan 618400 (CN)
(74) Representative: Kransell & Wennborg KB
(86) International application number: PCT/CN2023/097523
(87) International publication number: WO 2024/007772

(57) **Abstract**

A method for reducing the oligomer content in a fish oil, comprising: adding an antioxidant to the fish oil; after dissolution, processing the fish oil by means of distillation until 1-5% of the fish oil remains; and discarding the remaining fish oil. The fish oil obtained by means of distillation is a fish oil with a reduced oligomer content. The processing method requires simple equipment, is easy to operate, involves controllable reactions, and is conducive to industrial production.

## Description

### Cross-reference to Related Applications

The present disclosure claims priority to the Chinese patent application CN202210802874.3 filed with the Chinese Patent Office on July 7, 2022, and entitled "Method for Reducing Oligomer Content in Fish Oil", the contents of which are incorporated herein by reference in entirety.

### Technical Field

The present disclosure relates to the technical field of medicaments, and specifically to a method for reducing an oligomer content in fish oil.

### Background Art

Fish oil is a fat extracted from fatty fish, including: mackerel, tuna, salmon, sturgeon, anchovy, sardine, herring, trout, etc. The fish oil is rich in ω-3 polyunsaturated fatty acids, primarily eicosapentaenoic acid (EPA) and docosahexaenoic acid (DHA), while EPA and DHA each have different application fields: EPA is mainly used to lower blood fat, lower blood pressure, and prevent atherosclerosis; and DHA is mainly used to enhance memory and prevent myopia.

As the fish oil contains polyunsaturated fatty acids, during production, processing, storage and transportation of the fish oil, unsaturated carbon-carbon double bonds are highly prone to polymerization reaction to generate oligomers. As impurities in fish oil products, oligomers have been documented to potentially harm the human nervous system, and degradation of oligomers in the human body also will produce toxic small molecules. The European Pharmacopoeia and the United States Pharmacopoeia require that oligomers in fish oil are less than 1.0%.

However, a method that can reduce oligomers in fish oil disclosed in the prior art CN108911981A is: reacting fish oil with a catalyst and a low carbon alcohol solution containing glycerol, and then distilling reaction product, and performing adsorption treatment on the distilled product with an adsorbent, so as to obtain the fish oil with a reduced content of oligomers. This method needs to introduce catalysts such as sodium hydroxide, potassium hydroxide, calcium hydroxide, sodium methoxide and sodium ethoxide and adsorbents such as aluminum oxide, silica gel, activated carbon and amino silica gel, but residual catalysts and adsorbents are not treated.

### Summary

The present disclosure provides a method for reducing a content of oligomers in fish oil, wherein the method includes: to the fish oil adding an antioxidant, after dissolving, performing a distillation treatment until 1%-5% of the fish oil is left, and then discarding undistilled fish oil, and the fish oil obtained by the distillation is the fish oil with a reduced content of oligomers.

Optionally, the fish oil includes ethyl ester fish oil.

Optionally, an amount of the antioxidant added is 0.1%∼1% of a mass of the fish oil.

Optionally, the antioxidant includes at least one of tocopherol, tea polyphenol, flavonoids, butyl hydroxyanisole, dibutyl hydroxyl toluene and tert-butylhydroquinone.

Optionally, the antioxidant includes tocopherol.

Optionally, the tocopherol is at least one selected from the group consisting of mixed tocopherol concentrate, α-tocopherol, β-tocopherol, γ-tocopherol and δ-tocopherol.

Optionally, the dissolving step is carried out under a nitrogen-charging condition.

Optionally, the distillation treatment is vacuum distillation.

Optionally, a vacuum pressure of the vacuum distillation is 0~100 Pa.

Optionally, a temperature of the vacuum distillation is 150 °C~250 °C.

Optionally, the method further includes repeating the distillation treatment on the fish oil obtained by the distillation until the fish oil is free of oligomer.

Optionally, the distillation treatment is repeated for 1-5 times.

### Brief Description of Drawings

In order to more clearly illustrate technical solutions of examples of the present disclosure, drawings which need to be used in the examples will be introduced briefly below. It should be understood that the drawings below merely show some examples of the present disclosure, and therefore should not be considered as limitation to the scope. Those ordinarily skilled in the art still could obtain other relevant drawings according to these drawings, without using any inventive efforts.
FIG. 1 is a detection graph of ingredients of fish oil obtained in Example 1;
FIG. 2 is a detection graph of ingredients of fish oil obtained in Comparative Example 1;
FIG. 3 is a detection graph of ingredients of fish oil obtained in Comparative Example 2; and
FIG. 4 is a detection graph of ingredients of fish oil obtained in Comparative Example 3.

### Detailed Description of Embodiments

In order to make objectives, technical solutions and advantages of the embodiments and examples of the present disclosure clearer, technical solutions in the embodiments and examples of the present disclosure will be described clearly and completely below. Where no specific conditions are specified in the embodiments and examples, they are carried out under normal conditions or conditions recommended by manufacturers. Where manufacturers of reagents or apparatuses used are not specified, they are all conventional products commercially available.

Fish oil has health benefits of resisting inflammation, regulating blood lipids, etc. due to its richness in EPA and DHA, but as it contains polyunsaturated fatty acids, during production, processing, storage and transportation of the fish oil, unsaturated carbon-carbon double bonds are highly prone to polymerization reaction to generate oligomers. As used herein, "oligomer" may be a substance having a molecular weight greater than about 356, for example, oligomers include ethyl ester, diglyceride, monoglyceride, etc., whose presence may damage the human nervous system, and degradation of oligomers in the human body also will produce toxic small molecules. The inventors of the present disclosure have found through a series of inventive efforts that by adding a certain amount of antioxidant to the fish oil and performing distillation treatment, the content of oligomers in the fish oil may be reduced or even completely removed.

On this basis, the present disclosure provides a method for reducing a content of oligomers in fish oil, which includes the following steps: to the fish oil adding an antioxidant, after stirring and dissolving, performing distillation treatment until 1%-5% (mass percentage) of the fish oil is left, and discarding undistilled fish oil, and the fish oil obtained by the distillation is the fish oil with reduced oligomers. Optionally, the distillation is performed until a remaining amount of the fish oil may be 1%, 1.5%, 2.0%, 2.5%, 3.0%, 3.5%, 4.0%, 4.5%, 5.0%, or an interval value between any two of the above endpoints.

There are two types of fish oil: TG type (triglyceride type) and EE type (ethyl ester type). Triglyceride (TG) consists of three fatty acid molecules attached to a glycerol backbone. Actually, such molecular structure constitutes fat and grease in animals and plants. Most of omega-3 (ω-3) fatty acids existing in fish are of the TG type, that is, the triglyceride type fish oil is natural fish oil. The ethyl ester (EE) type consists of fatty acids attached to one ethanol molecule, and is obtained by cutting fatty acids with the aid of a catalyst and then linking the fatty acids to the ethanol molecule. Generally, the ethyl ester type is not naturally occurring, but is chemically synthesized.

In some embodiments, the fish oil to which the present disclosure is directed is ethyl ester type fish oil.

In some embodiments, the antioxidant used in the present disclosure may be tocopherol, and also may be tea polyphenol (TP), flavonoids, butyl hydroxyanisole (BHA), dibutyl hydroxyl toluene (BHT) and tert-butylhydroquinone (TBHQ) and other common food antioxidants.

Tocopherols are hydrolysis products of vitamin E. Natural tocopherols are all D-tocopherols (dextrorotatory), and have eight isomers such as α, β, Y, and δ. Tocopherols increase antioxidant effect of cells, and maintain and promote reproductive function, also have a certain anti-aging effect, and can improve lipid metabolism, prevent arteriosclerosis, and lower blood lipids. In the food field, the tocopherols are widely applied to full-fat milk powder, cream or margarine, meat products, aquatic processed products, dehydrated vegetables, fruit juice beverages, frozen foods, convenience foods, etc., and particularly, the tocopherols are of important significance as antioxidants and nutritional enhancers for baby foods, therapeutic foods, fortified foods, etc. In the medical field, the tocopherols are commonly used for the prevention of habitual abortion and threatened abortion, and for the treatment of climacteric syndrome, and are also used for adjuvant treatment of hepatitis, cirrhosis, and myodystrophy.

In some embodiments, the tocopherol may be mixed tocopherol concentrate (i.e., a mixture of various isomers of natural tocopherol), and also may be any one of α-tocopherol, β-tocopherol, γ-tocopherol and δ-tocopherol.

In some embodiments, an amount of the tocopherol added is 0.1%-1% of a mass of the fish oil.

Optionally, the amount of the tocopherol added may be, for example, 0.2%-0.9%, 0.3%-0.8% or 0.4%-0.7%, such as 0.1%, 0.2%, 0.3%, 0.4%, 0.5%, 0.6%, 0.7%, 0.8%, 0.9% or 1.0%, of the mass of the fish oil, and also may be any value between adjacent mass fractions in the above, or an interval value between any two of the above endpoints.

In some embodiments, the stirring and dissolving in the present disclosure are carried out under a nitrogen-charging condition.

Stirring to make the antioxidant dissolve in the fish oil under the nitrogen-charging condition after adding the antioxidant is to prevent loss or oxidation of certain beneficial ingredients in products caused by processing in an air environment, which affects the quality of the product.

In some embodiments, the distillation treatment is vacuum distillation.

The vacuum distillation is carried out under reduced pressure, and is used to separate substances that are easy to decompose when heated to a boiling point under atmospheric pressure. By using the method of vacuum distillation, a distillation temperature may be reduced and separation efficiency may be improved.

In some embodiments, vacuum pressure of the above vacuum distillation is 0~100 Pa. Optionally, the vacuum pressure of the vacuum distillation may be 0.1 Pa, 1 Pa, 10 Pa, 30 Pa, 50 Pa, 70 Pa, 90 Pa or 100 Pa, or an interval value between any two of the above endpoints.

In some embodiments, a temperature of the above vacuum distillation is 150 °C~250 °C. Optionally, the temperature of the above vacuum distillation may be 155 °C, 160 °C, 170 °C, 180 °C, 190 °C, 200 °C, 210 °C, 220 °C, 230 °C, 240 °C or 245 °C, or an interval value between any two of the above endpoints.

In some embodiments, the above method further includes repeating the vacuum distillation on the fish oil obtained by the distillation until the fish oil is free of oligomers.

According to the method for reducing a content of oligomers in fish oil provided in the present disclosure, based on different oligomer contents in raw materials of the fish oil, and different remaining amounts of the oligomer content in the fish oil after a first time of vacuum distillation treatment, the vacuum distillation treatment may be repeated on the fish oil still containing oligomers after the first time of vacuum distillation treatment, until the oligomer content in the obtained fish oil is as low as possible, and even the fish oil is free of oligomers.

In some embodiments, the vacuum distillation treatment is repeated for 1-5 times.

Optionally, the vacuum distillation treatment may be repeated for 1 time, 2 times, 3 times, 4 times or 5 times.

The method for reducing a content of oligomers in fish oil provided in the present disclosure overcomes the damage to health of the human body caused by the presence of oligomers in the fish oil, and the removal method will not introduce harmful substances into fish oil products.

By the method for reducing a content of oligomers in fish oil provided in the present disclosure, only by adding the antioxidant and then performing the distillation treatment, the oligomers in the fish oil can be reduced to the lowest possible level, so as to obtain the fish oil products without oligomers, and meanwhile, the adopted food antioxidants will not damage the health of the human body, further ensuring safety of the fish oil products. Moreover, the treatment method according to the present disclosure is simple in equipment requirement, simple and convenient to operate, controllable in reaction and easy to realize industrial production.

### Examples

The features and performances of the present disclosure are further described in detail below in combination with examples.

### Example 1

The present disclosure provides a method for reducing oligomers in fish oil, including the following steps:
(1) to oligomer-containing fish oil adding mixed tocopherol concentrate, wherein an amount of the mixed tocopherol concentrate added was 0.1% of a mass of the fish oil, and stirring and dissolving at a normal temperature under protection of nitrogen;
(2) performing distillation on the above fish oil after the dissolution under a vacuum condition, wherein a vacuum pressure was 0.1 Pa, and a distillation temperature was 150 °C;
(3) distilling the above fish oil until 1-5% of the fish oil was left, and discarding the undistilled fish oil; and
(4) repeating steps (2) and (3) on the fish oil obtained by the distillation for 3 times.

### Example 2

The present disclosure provides a method for reducing oligomers in fish oil, including the following steps:
(1) to oligomer-containing fish oil adding mixed tocopherol concentrate, wherein an amount of the mixed tocopherol concentrate added was 0.5% of a mass of the fish oil, and stirring and dissolving at a normal temperature under protection of nitrogen;
(2) performing distillation on the above fish oil after the dissolution under a vacuum condition, wherein a vacuum pressure was 0.1 Pa, and a distillation temperature was 150 °C;
(3) distilling the above fish oil until 1-5% of the fish oil was left, and discarding the undistilled fish oil; and
(4) repeating steps (2) and (3) on the fish oil obtained by the distillation for 3 times.

### Example 3

The present disclosure provides a method for reducing oligomers in fish oil, including the following steps:
(1) to oligomer-containing fish oil adding mixed tocopherol concentrate, wherein an amount of the mixed tocopherol concentrate added was 1% of a mass of the fish oil, and stirring and dissolving at a normal temperature under protection of nitrogen;
(2) performing distillation on the above fish oil after the dissolution under a vacuum condition, wherein a vacuum pressure was 0.1 Pa, and a distillation temperature was 150 °C;
(3) distilling the above fish oil until 1-5% of the fish oil was left, and discarding the undistilled fish oil; and
(4) repeating steps (2) and (3) on the fish oil obtained by the distillation for 3 times.

### Example 4

The present disclosure provides a method for reducing oligomers in fish oil, including the following steps:
(1) to oligomer-containing fish oil adding mixed tocopherol concentrate, wherein an amount of the mixed tocopherol concentrate added was 0.5% of a mass of the fish oil, and stirring and dissolving at a normal temperature under protection of nitrogen;
(2) performing distillation on the above fish oil after the dissolution under a vacuum condition, wherein a vacuum pressure was 100 Pa, and a distillation temperature was 150 °C;
(3) distilling the above fish oil until 1-5% of the fish oil was left, and discarding the undistilled fish oil; and
(4) repeating steps (2) and (3) on the fish oil obtained by the distillation for 3 times.

### Example 5

The present disclosure provides a method for reducing oligomers in fish oil, including the following steps:
(1) to oligomer-containing fish oil adding mixed tocopherol concentrate, wherein an amount of the mixed tocopherol concentrate added was 0.5% of a mass of the fish oil, and stirring and dissolving at a normal temperature under protection of nitrogen;
(2) performing distillation on the above fish oil after the dissolution under a vacuum condition, wherein a vacuum pressure was 0.1 Pa, and a distillation temperature was 250 °C;
(3) distilling the above fish oil until 1-5% of the fish oil was left, and discarding the undistilled fish oil; and
(4) repeating steps (2) and (3) on the fish oil obtained by the distillation for 3 times.

### Comparative Example 1

Compared with Example 1, the present comparative example differs in that no antioxidant was added. Steps thereof are as follows:
(1) performing distillation on oligomer-containing fish oil under a vacuum condition, wherein a vacuum pressure was 0.1 Pa, and a distillation temperature was 150 °C;
(2) distilling the above fish oil until 1-5% of the fish oil was left, and discarding the undistilled fish oil; and
(3) repeating steps (1) and (2) on the fish oil obtained by the distillation for 3 times.

### Comparative Example 2

Compared with Example 1, the present comparative example differs in that the amount of the antioxidant added was 1.1%. Steps thereof are as follows:
(1) to oligomer-containing fish oil adding mixed tocopherol concentrate, wherein an amount of the mixed tocopherol concentrate added was 1.1% of a mass of the fish oil, and stirring and dissolving at a normal temperature under protection of nitrogen;
(2) performing distillation on the above fish oil after the dissolution under a vacuum condition, wherein a vacuum pressure was 0.1 Pa, and a distillation temperature was 150 °C;
(3) distilling the above fish oil until 1-5% of the fish oil was left, and discarding the undistilled fish oil; and
(4) repeating steps (2) and (3) on the fish oil obtained by the distillation for 3 times.

### Comparative Example 3

A content of oligomers in fish oil was reduced by using a method in the prior patent document CN108911981A, and steps thereof are as follows:
(1) taking 100 g of fish oil ethyl ester, adding 0.5 g of sodium methoxide, and placing the same in a reaction kettle; adding 5 g of glycerol to 50 ml of anhydrous ethanol to dissolve, and placing the resultant in a constant-pressure dropwise addition device, introducing nitrogen into the reaction kettle, starting to stir, and heating, when the temperature reached 124 °C, adding glycerol-containing anhydrous ethanol solution dropwise at a rate of 0.17 ml/min, reacting for 7 hours, cooling, filtering, and washing with water, so as to obtain crude triglycerides;
(2) transferring materials obtained in step (1) into a distillation device after centrifugation, inserting a nitrogen pipe under a liquid level of the materials to charge nitrogen, gradually releasing vacuum while gradually reducing nitrogen flow rate, after the pressure was stable at 0.095 MPa, heating to 205 °C, distilling for 5 hours, and after the distillation was ended, cooling the materials to room temperature; and
(3) transferring materials obtained in step (2) to an adsorption device, charging nitrogen, adding 0.5 g of silica gel, stirring and adsorbing for 5 hours, and subjecting the resultant to filtration by a filter element and precision filtration, so as to obtain ω-3-fatty acid triglyceride for injection.

### Experimental Example

Ingredients of the fish oil prepared in Examples 1-5 and Comparative Examples 1-3 were detected for the content of oligomers in the fish oil, and detection results are as follows:

**Table 1 Detection Results of Content of Oligomers in Fish Oil**

| | Example 1 | Example 2 | Example 3 | Example 4 | Example 5 | Comparative Example 1 | Comparative Example 2 | Comparative Example 3 |
|---|---|---|---|---|---|---|---|---|
| Oligomer Content | Undetected | Undetected | Undetected | Undetected | Undetected | 0.1% | 0.4% | 0.3% |

From the detection results in Table 1, it may be seen that the fish oil in Comparative Examples 1-3 after the treatment still contained small amounts of oligomers, whereas none of the fish oil treated in Examples 1-5 had oligomers. Therefore, compared with Comparative Examples 1-3, Examples 1-5 can effectively remove the oligomer content in the fish oil by using the method disclosed in the present disclosure.

A method for detecting the oligomers is as follows.

The oligomer content in Examples 1-5 and Comparative Examples 1-3 was detected by high-performance liquid chromatography, wherein chromatography conditions were as follows.

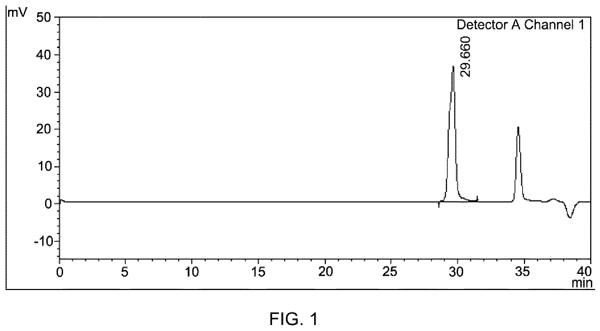

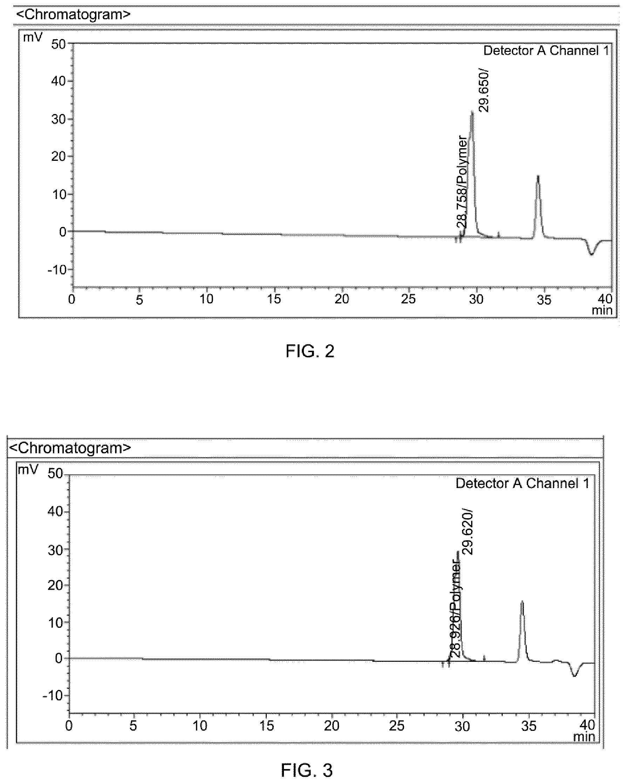

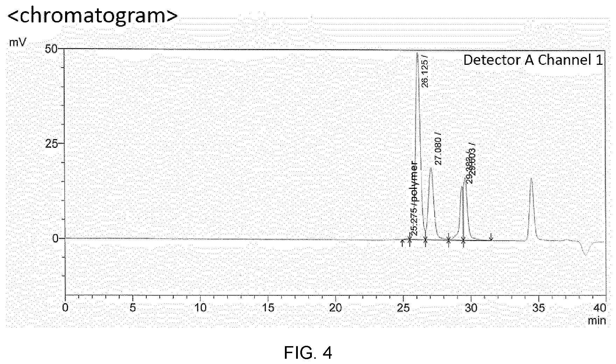

Chromatographic columns were gel columns containing styrene divinyl benzene, respectively:
column 1 (close to feeding end): 5 µm 500A, 300×7.8 mm 5 micron;
column 2 (middle): 5µm 100A, 300×7.8 mm 5 micron;
column 3 (close to detector): 5 µm 50A, 300×7.8 mm 5 micron;
a mobile phase in the high-performance liquid chromatography was tetrahydrofuran, a flow rate of the mobile phase was 0.8 mL/min, a sample size was 40 µL, concentration of sample was 5 mg/ml, a column temperature was 40 °C, isocratic elution was performed, and a differential refractive index detector was used for measurement (a temperature of the differential refractive index detector was 40 °C). Partial detection results are shown in FIGS. 1-4, in which FIG. 1 is a detection graph of ingredients of fish oil obtained by taking Example 1 as an example, FIG. 2 is a detection graph of ingredients of fish oil obtained in Comparative Example 1, FIG. 3 is a detection graph of ingredients of fish oil obtained in Comparative Example 2, and FIG. 4 is a detection graph of ingredients of fish oil obtained in Comparative Example 3.

It can be seen from FIG. 1 and FIGS. 2-4 that the fish oil treated in Example 1 substantially had no oligomers in the chromatogram, while the fish oil treated in Comparative Examples 1-3 still contained a small amount of oligomers.

In conclusion, after being treated by the method for reducing a content of oligomers in fish oil provided in the present disclosure, the oligomers in the fish oil can be reduced to the lowest possible level, and even the fish oil products without oligomers may be obtained.

The above-mentioned are only optional examples of the present disclosure, and are not used to limit the present disclosure. For those skilled in the art, various modifications and changes could be made to the present disclosure. Any modifications, equivalent substitutions, improvements and the like made within the spirit and principle of the present disclosure should be covered within the scope of protection of the present disclosure.

### Industrial Applicability

The present disclosure provides a method for reducing a content of oligomers in fish oil, wherein only by adding the antioxidant and then performing the distillation treatment, the oligomers in the fish oil can be reduced to the lowest possible level, so as to obtain the fish oil products without oligomers, and meanwhile, the adopted food antioxidants will not damage the health of the human body, further ensuring safety of the fish oil products. Moreover, the treatment method according to the present disclosure is simple in equipment requirement, simple and convenient to operate, controllable in reaction easy to realize industrial production, and therefore has excellent practical performance.

## Claims

1. A method for reducing a content of oligomers in fish oil, **characterized in that** the method comprises: to the fish oil adding an antioxidant, after dissolving, performing a distillation treatment until 1%-5% of the fish oil is left, and then discarding undistilled fish oil, and fish oil obtained by the distillation is fish oil with a reduced content of oligomers.

2. The method according to claim 1, wherein the fish oil comprises ethyl ester fish oil.

3. The method according to claim 1 or 2, wherein an amount of the antioxidant added is 0.1%~1 % of a mass of the fish oil.

4. The method according to claim 3, wherein the antioxidant comprises at least one of tocopherol, tea polyphenol, flavonoids, butyl hydroxyanisole, dibutyl hydroxyl toluene and tert-butylhydroquinone.

5. The method according to claim 3, wherein the antioxidant comprises tocopherol.

6. The method according to claim 4 or 5, wherein the tocopherol is at least one selected from the group consisting of mixed tocopherol concentrate, α-tocopherol, β-tocopherol, γ-tocopherol and δ-tocopherol.

7. The method according to any one of claims 1-6, wherein the dissolving step is carried out under a nitrogen-charging condition.

8. The method according to any one of claims 1-7, wherein the distillation treatment is vacuum distillation.

9. The method according to claim 8, wherein a vacuum pressure of the vacuum distillation is 0~100 Pa.

10. The method according to claim 8, wherein a temperature of the vacuum distillation is 150 °C~250 °C.

11. The method according to any one of claims 1-10, wherein the method further comprises repeating the distillation treatment on the fish oil obtained by the distillation until the fish oil is free of the oligomers; and preferably, the distillation treatment is repeated for 1-5 times.
